# EUROPÄISCHE PATENTANMELDUNG

(11) **EP 0 848 942 A1**
(43) Veröffentlichungstag der Anmeldung: **24.06.1998**
(21) Anmeldenummer: 97117209.3
(22) Anmeldetag: 04.10.1997
(51) Int. Cl.: A61K 7/13, D06P 3/08, D06P 1/32

(54) **Mittel und Verfahren zum Färben von Keratinfasern**

(30) Priorität: 20.12.1996 DE 19653292
(71) Anmelder: Wella Aktiengesellschaft, 64274 Darmstadt (DE)
(72) Erfinder: Braun, Hans-Jürgen, Dr., 3182 Überstorf (CH); Umbricht, Gisela, Dr., 1700 Fribourg (CH)

(57) **Zusammenfassung**

Mittel zum Färben von Keratinfasern, welches
(a) o-Benzochinone der Formel (I) 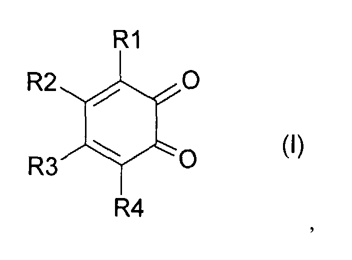 mit R¹, R², R³ und R⁴ unabhängig voneinander gleich Wasserstoff, Chlor, einer C₁- bis C₅- Alkylgruppe, einer C₁- bis C₅- Alkoxygruppe, einer C₁- bis C₂- Alkylendioxygruppe oder einer Aminogruppe -NR⁵R⁶, wobei R⁵ und R⁶ unabhängig voneinander Wasserstoff, eine Arylgruppe oder eine C₁- bis C₆- Alkylgruppe bedeuten, und
(b) mindestens eine Amino- oder Hydroxyverbindung der Formel (II)

   X-A-Y (II),

   wobei X eine Nitrogruppe, eine Cyanogruppe oder eine Sulfonatgruppe bedeutet, Y eine Hydroxygruppe oder Aminogruppe -NR^{a}R^{b}, mit R^{a} und R^{b} unabhängig voneinander gleich Wasserstoff oder einer C₁- bis C₄-Alkylgruppe bedeutet und A für eine substituierte oder unsubstituierte geradkettige, zyklische oder verzweigte C₁- bis C₆- Alkylgruppe oder einen substituierten oder unsubstituierten aromatischen Heterozyklus oder Carbozyklus steht, enthält.

## Beschreibung

Gegenstand der Erfindung sind Mittel zum Färben von Keratinfasern, welche o-Benzochinone in Kombination mit bestimmten Amino- oder Hydroxyverbindungen enthalten, sowie ein Verfahren zur Färbung von Keratinfasern unter Verwendung dieser Mittel.

Für das Färben von keratinhaltigen Fasern, zum Beispiel Haaren, Wolle oder Pelzen, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Mit Oxidationsfarbstoffen lassen sich zwar intensive Färbungen mit guten Echtheitseigenschatten erzielen, die Entwicklung der Farbe geschieht jedoch unter dem Einfluß von starken Oxidationsmitteln, wie zum Beispiel Wasserstoffperoxid, was häufig Schädigungen der Faser zur Folge hat. Direktziehende Farbstoffe werden unter schonenderen Bedingungen appliziert, ihr Nachteil liegt jedoch darin, daß die Färbungen häufig nur über unzureichende Echtheitseigenschaften verfügen.

Gemäß der DE-OS 43 35 624 sollen Färbemittel auf der Basis von p-Benzochinonen, bei denen auf den Einsatz von zusätzlichen Oxidationsmitteln verzichtet werden kann, hier eine Alternative darstellen.

Das in der DE-OS 43 35 624 beschriebene Färbeverfahren ist jedoch in der Praxis nur schwer durchführbar, da die Färbemischung vor der Anwendung auf Siedetemperatur erhitzt und anschließend filtriert werden muß, und führt zudem häufig zu unbefriedigenden Färberesultaten.

Es bestand daher die Aufgabe, nicht-oxidative Färbemittel für Keratinfasern zur Verfügung zu stellen, welche leicht anwendbar sind und intensive Färbungen ermöglichen.

Hierzu wurde nunmehr gefunden, daß durch ein Mittel zum Färben von Keratinfasern, wie zum Beispiel menschlichen Haaren, Wolle oder Pelzen, das (a) o-Benzochinone sowie (b) bestimmte Amino- oder Hydroxyverbindungen, welche mit stark elektronenziehenden Substituenten substituiert sind, enthält, die gestellte Aufgabe in hervorragender Weise gelöst wird.

Gegenstand der Erfindung ist ein Mittel zum Färben von Keratinfasern, insbesondere menschlichen Haaren, enthaltend
(a) o-Benzochinone der Formel (I) 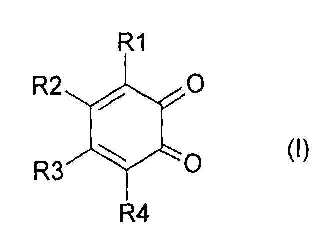 mit R¹, R², R³ und R⁴ unabhängig voneinander gleich Wasserstoff, Chlor, einer C₁- bis C₅- Alkylgruppe, einer C₁- bis C₅- Alkoxygruppe, einer C₁- bis C₂- Alkylendioxygruppe oder einer Aminogruppe -NR⁵R⁶, wobei R⁵ und R⁶ unabhängig voneinander Wasserstoff, eine Arylgruppe oder eine C₁- bis C₆- Alkylgruppe bedeuten, und
(b) mindestens eine Amino- oder Hydroxyverbindung der Formel (II)

   X-A-Y (II),

   wobei X eine Nitrogruppe, eine Cyanogruppe oder eine Sulfonatgruppe bedeutet, Y eine Hydroxygruppe oder Aminogruppe -NR^{a}R^{b}, mit R^{a} und R^{b} unabhängig voneinander gleich Wasserstoff oder einer C₁- bis C₄- Alkylgruppe bedeutet und A für eine substituierte oder unsubstituierte geradkettige, zyklische oder verzweigte C₁- bis C₆- Alkylgruppe oder einen substituierten oder unsubstituierten aromatischen Heterozyklus oder Carbozyklus steht.

Bevorzugt sind solche Färbemittel, bei denen die Verbindung der Formel (II) ausgewählt ist aus
(1) Anilinderivaten der Formel (IIa) 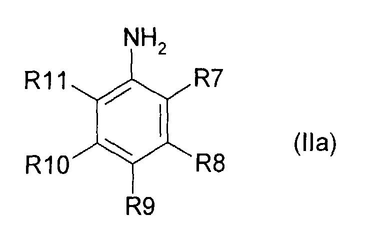 wobei mindestens einer der Reste R⁷, R⁸, R⁹, R¹⁰ und R¹¹ eine Nitrogruppe, eine Cyanogruppe oder eine Sulfonatgruppe bedeutet, und die übrigen Reste R⁷ bis R¹¹ unabhängig voneinander Wasserstoff, Chlor, eine Hydroxygruppe, eine Aminogruppe, eine C₁- bis C₅- Alkylgruppe, eine C₁- bis C₅- Hydroxyalkylgruppe, eine C₁- bis C₅-Alkoxygruppe, eine C₂- bis C₅-Hydroxyalkoxygruppe oder eine Carboxygruppe bedeuten,
(2) Phenolderivaten der Formel (IIb) 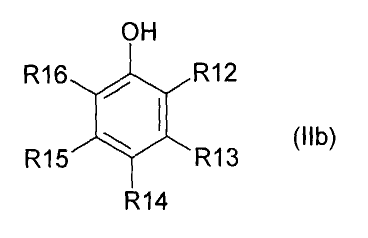 wobei mindestens einer der Reste R¹²,R¹³,R¹⁴, R¹⁵ und R¹⁶ eine Nitrogruppe, eine Cyanogruppe oder eine Sulfonatgruppe bedeutet, und die übrigen Reste R¹² bis R¹⁶ unabhängig voneinander Wasserstoff, Chlor, eine Hydroxygruppe, eine C₁- bis C₅-Hydroxyalkylgruppe, eine C₁- bis C₅-Alkoxygruppe, eine C₂- bis C₅-Hydroxyalkoxygruppe oder eine Carboxylgruppe darstellen,
(3) mehrkernigen aromatischen Heterozyklen oder Isozyklen, die mit mindestens einer Amino- oder Hydroxygruppe und mindestens einer Nitrogruppe, Cyanogruppe oder Sulfonatgruppe substituiert sind,
(4) fünf- bis sechsgliedrigen Heterozyklen mit 1 bis 3 Stickstoffatomen, die mit mindestens einer Amino- oder Hydroxygruppe und mindestens einer Nitrogruppe, Cyanogruppe oder Sulfonatgruppe substituiert sind,
(5) offenkettigen gesättigten oder ungesättigten Verbindungen, die mindestens zwei Aminogruppen oder je eine Aminogruppe und eine Hydroxygruppe sowie mindestens eine Nitrogruppe, Cyanogruppe oder Sulfonatgruppe aufweisen.

Die vorgenannten Verbindungen können auch in Form ihrer physiologisch verträglichen Säureadditionsverbindungen, zum Beispiel in Form der Chloride, Sulfate oder Laktate, beziehungsweise bei Phenolen oder Carboxylaten in Form ihrer Alkalisalze, Erdalkalisalze oder quaternären Ammoniumsalze vorliegen.

Als besonders bevorzugte Verbindungen der Formel (II) sind 2-Nitroanilin, 3-Nitroanilin, 4-Nitro-o-phenylendiamin, 2-Amino-5-nitrophenol, 1,4-Diamino-2-nitrobenzol, 2-Amino-3,5-dinitrophenol, Pikraminsäure, 1,2-Diamino-3-nitrobenzol, 2-Cyanoanilin, Diaminomaleinsauredinitril, 1-Amino-2-naphthol-4-sulfonsäure, 2-Aminophenol-4-sulfonsäure, 2-(4,5,6-Trihydroxy-3-oxo-3H-xanthen-9-yl)-benzolsulfonäure (Pyrogallolrot) und p-Phenylendiamin-sulfonsäure zu nennen.

Geeignete o-Benzochinone der Formel (I) sind zum Beispiel 4,5-Dimethoxy-1,2-benzochinon, 4,5-Methylendioxy-1,2-benzochinon, 4,5-Dianilino-1,2-benzochinon, o-Chloranil und 3,5-Di-(tert-butyl)-1,2-benzochinon.

Das o-Benzochinon der Formel (I) ist in dem erfindungsgemäßen Färbemittel in einer Menge von 0,02 bis 3 Gewichtsprozent, insbesondere einer Menge von 0,03 bis 1 Gewichtsprozent, enthalten, während die Verbindungen der Formel (II) in einer Menge von 0,03 bis 6 Gewichtsprozent, insbesondere einer Menge von 0,03 bis 1 Gewichtsprozent, eingesetzt wird.

Die Zubereitungsform des hier beschriebenen Haarfärbemittels kann beispielsweise die einer Lösung, insbesondere einer wäßrigen oder wäßrig-alkoholischen Lösung, sein. Bevorzugte Zubereitungsformen sind weiterhin die einer Creme, eines Geles, einer Emulsion oder die einer tensidhaltigen schäumenden Zusammensetzung, beispielsweise eines Shampoos.

Der pH-Wert des Färbemittels liegt im Bereich von 3 bis 10,5, insbesondere bei einem pH-Wert von 4 bis 9, wobei die Einstellung des alkalischen pH-Wertes mit Ammoniak oder mit organischen Aminen, wie beispielsweise Monoethanolamin oder Triethanolamin, vorgenommen werden kann, während für die Einstellung des sauren pH-Wertes organische Säuren, wie zum Beispiel Milchsäure, Zitronensäure oder Essigsäure, verwendet werden können.

Selbstverständlich kann das vorstehend beschriebene Haarfärbemittel gegebenenfalls weitere, für Haarfärbemittel übliche Zusätze, wie zum Beispiel Konservierungsstoffe und Parfümöle, Lösungsmittel wie Wasser, niedere aliphatische Alkohole, beispielsweise Ethanol, Propanol und Isopropanol, oder Glykole wie Glycerin und 1,2-Propylenglykol, weiterhin

Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide, oxethylierte Fettsäureester, ferner Verdicker wie höhere Fettalkohole, Stärke oder Cellulosederivate, weiterhin Weichmacher, Vaseline, Paraffinöl und Fettsäuren sowie außerdem Pflegestoffe, wie kationische Harze, Lanolinderivate, Cholesterin, Vitamine, Pantothensäure und Betain, enthalten. Die erwähnten Bestandteile werden in den für solche Zwecke üblichen Mengen verwendet, zum Beispiel die Netzmittel und Emulgatoren in Konzentrationen von 0,5 bis 30 Gewichtsprozent, die Verdicker in einer Menge von 0,1 bis 25 Gewichtsprozent und die Pflegestoffe in einer Konzentration von 0,1 bis 5,0 Gewichtsprozent.

Die beiden Verbindungen der Formeln (I) und (II) können getrennt oder zusammen, entweder in wasserfreier Form oder in Form einer gebrauchsfertigen Formulierung konfektioniert sein.

Vorzugsweise werden die beiden Verbindungen der Formeln (I) und (II) jedoch getrennt in Form einer 2-Komponentenverpackung abgepackt, wobei beide Komponenten sowohl in wasserfreier Form als auch in Form einer wäßrigen oder wäßrig-alkoholischen Zubereitung vorliegen können. Sofern die beiden Komponenten in wasserfreier Form vorliegen, werden sie vor der Anwendung in Wasser oder einer geeigneten wäßrigen oder wäßrig-alkoholischen Kosmetikgrundlage gelöst, wobei die beiden Komponenten in getrennten Lösungen oder in einer gemeinsamen Lösung vorliegen können.

Zum Haarefärben wird das erfindungsgemäße Färbemittel in einer für die Haarfärbung ausreichenden Menge auf das Haar aufgebracht, dort für etwa 20 bis 30 Minuten bei einer Temperatur von etwa 20 bis 40 Grad Celsius belassen, und anschließend mit Wasser ausgespült oder mit einem handelsüblichen Haarshampoo ausgewaschen. Abschließend werden die Haare getrocknet.

Falls das erfindungsgemäße Mittel in Form einer 2-Komponentenverpackung vorliegt, ist es möglich, die Verbindungen der Formeln (I) und (II) entweder unmittelbar vor der Anwendung miteinander zu vermischen und die erhaltene Zubereitung auf das Haar aufzubringen oder aber die beiden Verbindungen der Formeln (I) und (II) nacheinander auf das Haar aufzubringen, wobei zwischen dem Auftragen der die Verbindung der Formel (I) enthaltenden Zubereitung und dem Auftragen der die Verbindung der Formel (II) enthaltenden Zubereitung ein Zeitabstand von etwa 2 bis 5 Minuten liegen kann.

Das erfindungsgemäße Färbemittel ermöglicht eine einfache und intensive Färbung von Keratinfasern, insbesondere menschlichen Haaren, mit einer ausgezeichneten Stabilität gegenüber Lichteinwirkung und Shampoonieren.

Mit dem erfindungsgemäßen Färbemittel sind eine Vielzahl von Farbnuancen, beispielsweise gelbe, orange, braune, rote, violette oder schwarze Farbtöne, möglich.

Die folgenden Beispiele sollen den Gegenstand der Erfindung näher erläutern, ohne diesen hierauf zu beschränken.

### Beispiele

### Beispiele 1 bis 13: Haarfärbemittel

In allen Beispielen werden Färbelösungen der folgenden Zusammensetzung verwendet:

### Färbelösung (I)

| | |
|---|---|
| ***Y ·*** 2,5 mmol | o-Benzochinon der Formel (I) |
| 10,0 g | Laurylalkoholdiglykolethersulfat-Natriumsalz (28prozentige wäßrige Lösung) |
| 10,0 g | Isopropanol |
| ad 100,0 g | Wasser, vollentsalzt |

### Färbelösung (II)

| | |
|---|---|
| ***Z ·*** 2,5 mmol | Verbindung der Formel (II) |
| 10,0 g | Laurylalkoholdiglykolethersulfat-Natriumsalz (28prozentige wäßrige Lösung) |
| 10,0 g | Isopropanol |
| ad 100,0 g | Wasser, vollentsalzt |

Die Faktoren ***Y*** und ***Z*** entsprechen den in der nachfolgenden Tabelle 1 angegebenen Verhältniszahlen.

Die Einstellung des gewünschten sauren pH-Wertes erfolgt durch Zusatz von 10 Gramm Milchsäure.

Die Färbelösung (I) wird jeweils bei Raumtemperatur (20 bis 25 Grad Celsius) gleichmäßig auf das zu färbende Haar aufgetragen. Nach einer Einwirkungszeit von 5 Minuten wird sodann die Färbelösung (II) (sowie gegebenenfalls eine 10%ige Milchsäurelösung) gleichmäßig auf das Haar aufgetragen.

Nach einer weiteren Einwirkungszeit von 20 Minuten bei 40 Grad Celsius werden die Haare mit lauwarmem Wasser gespült und getrocknet.

Die mit den verschiedenen Färbelösungen erhaltenen Färbeergebnisse sind in der nachfolgenden Tabelle 1 zusammengefaßt.

Die in den vorliegenden Beispielen ermittelten **Lab**-Farbmesswerte wurden mit einem Farbmessgerät der Firma Minolta, Typ Chromameter II, ermittelt.

Hierbei steht der L-Wert für die Helligkeit (das heißt je geringer der L-Wert ist, umso größer ist die Farbintensität), während der a-Wert ein Maß für den Rotanteil ist (das heißt je größer der a-Wert ist, umso größer ist der Rotanteil). Der b-Wert ist ein Maß für den Blauanteil der Farbe, wobei der Blauanteil umso größer ist, je negativer der b-Wert ist.

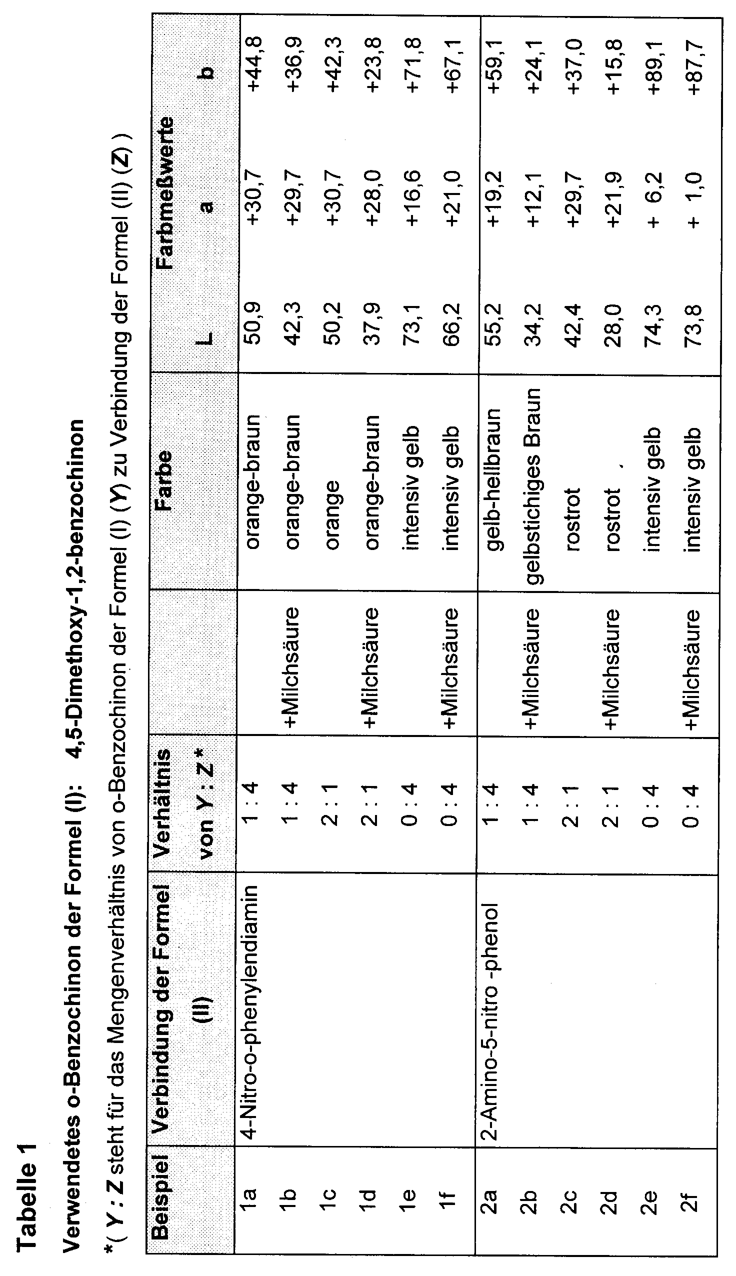

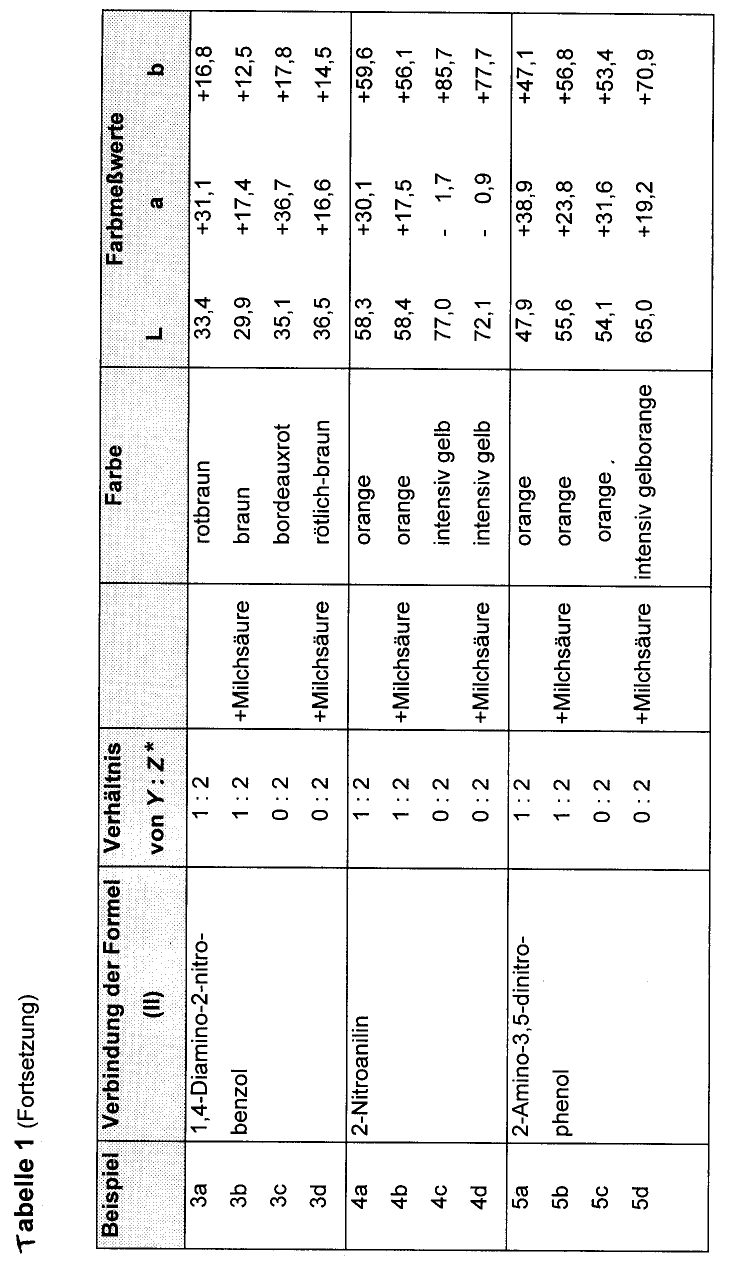

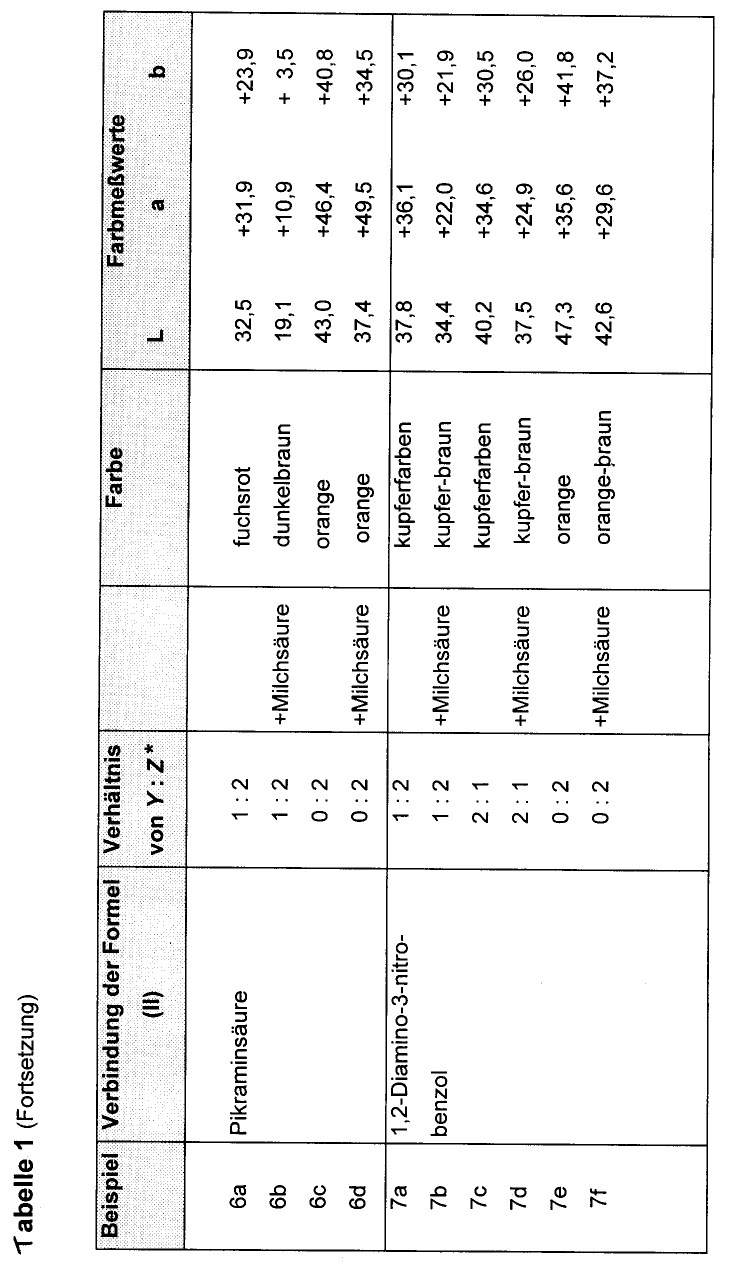

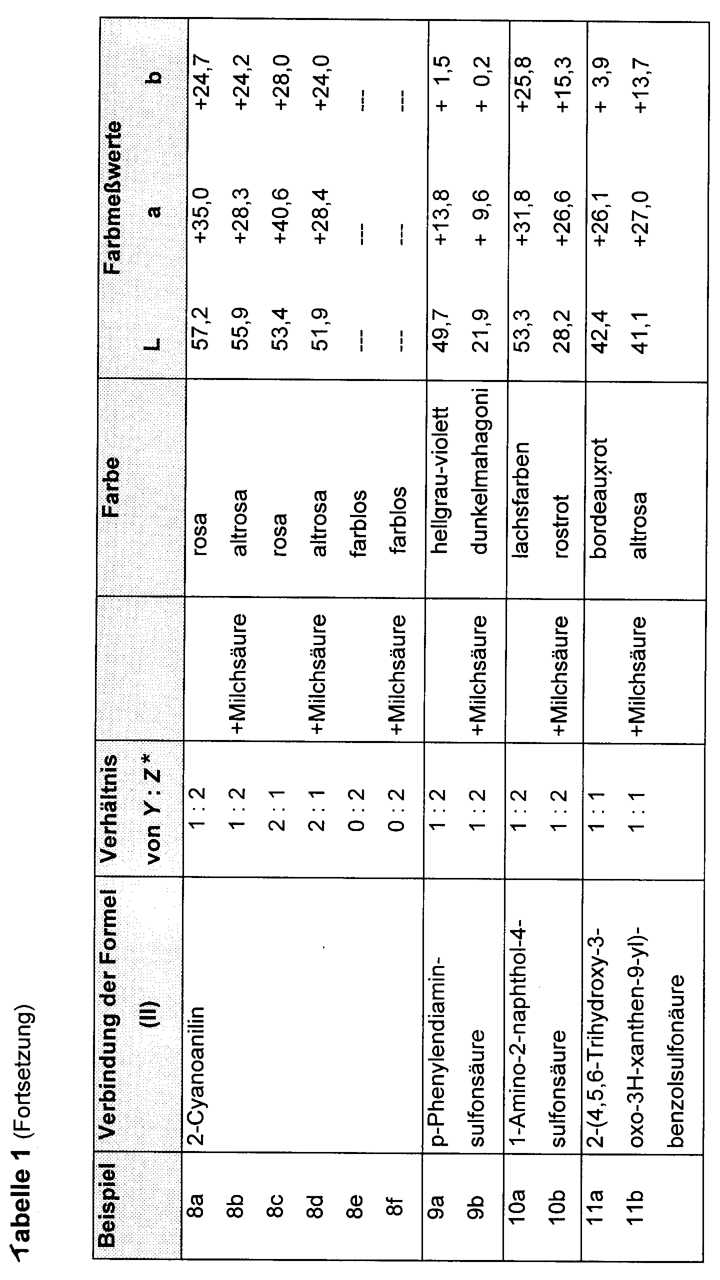

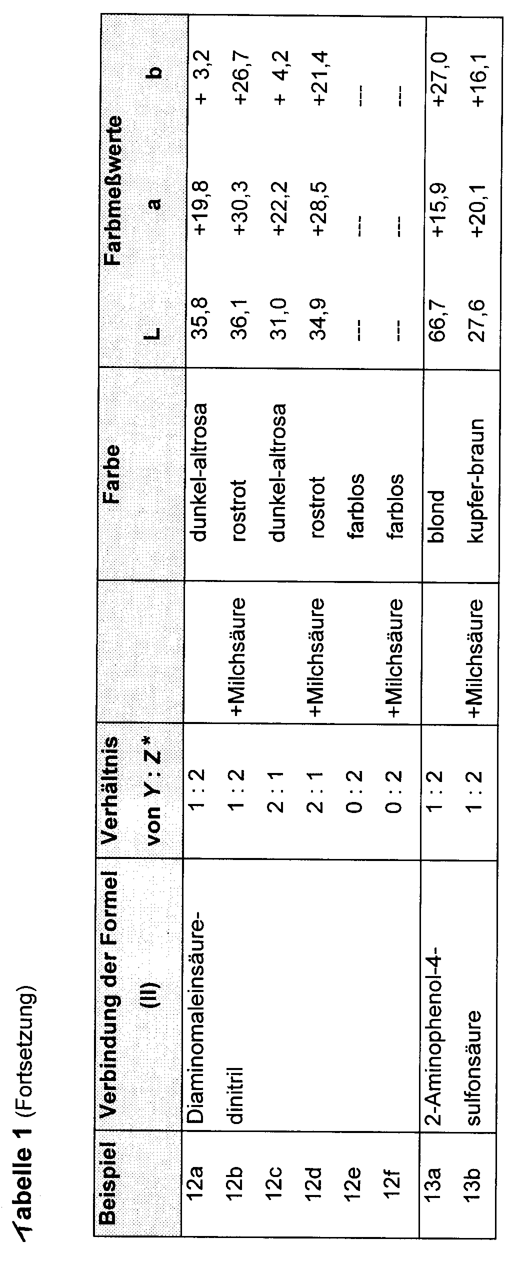

## Patentansprüche

1. Mittel zum Färben von Keratinfasern, welches dadurch gekennzeichnet ist, daß es
(a) o-Benzochinone der Formel (I) 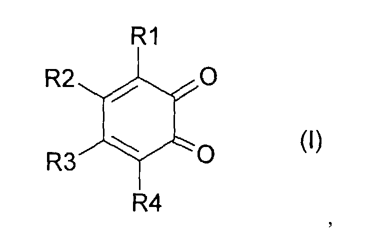 mit R¹, R², R³ und R⁴ unabhängig voneinander gleich Wasserstoff, Chlor, einer C₁- bis C₅- Alkylgruppe, einer C₁- bis C₅- Alkoxygruppe, einer C₁- bis C₂- Alkylendioxygruppe oder einer Aminogruppe -NR⁵R⁶, wobei R⁵ und R⁶ unabhängig voneinander Wasserstoff, eine Arylgruppe oder eine C₁- bis C₆- Alkylgruppe bedeuten, und
(b) mindestens eine Amino- oder Hydroxyverbindung der Formel (II)
X-A-Y (II),
wobei X eine Nitrogruppe, eine Cyanogruppe oder eine Sulfonatgruppe bedeutet, Y eine Hydroxygruppe oder Aminogruppe -NR^{a}R^{b}, mit R^{a} und R^{b} unabhängig voneinander gleich Wasserstoff oder einer C₁- bis C₄- Alkylgruppe bedeutet und A für eine substituierte oder unsubstituierte geradkettige, zyklische oder verzweigte C₁- bis C₆- Alkylgruppe oder einen substituierten oder unsubstituierten aromatischen Heterozyklus oder Carbozyklus steht, enthält.

2. Mittel nach Anspruch 1, dadurch gekennzeichnet, daß die Verbindung der Formel (II) ausgewählt ist aus
(1) Anilinderivaten der Formel (IIa) 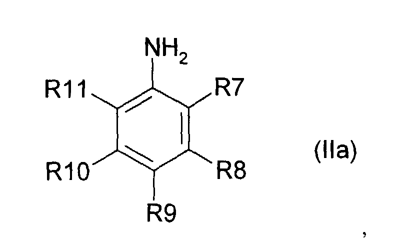 wobei mindestens einer der Reste R⁷, R⁸, R⁹, R¹⁰ und R¹¹ eine Nitrogruppe, eine Cyanogruppe oder eine Sulfonatgruppe bedeutet, und die übrigen Reste R⁷ bis R¹¹ unabhängig voneinander Wasserstoff, Chlor, eine Hydroxygruppe, eine Aminogruppe, eine C₁- bis C₅- Alkylgruppe, eine C₁- bis C₅- Hydroxyalkylgruppe, eine C₁- bis C₅-Alkoxygruppe, eine C₂- bis C₅-Hydroxyalkoxygruppe oder eine Carboxygruppe bedeuten,
(2) Phenolderivaten der Formel (IIb) 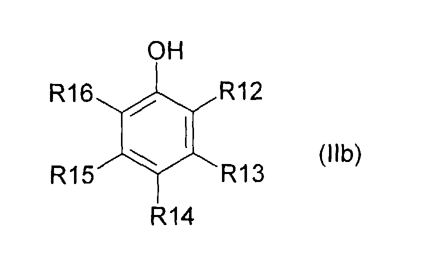 wobei mindestens einer der Reste R¹²,R¹³`R¹⁴, R¹⁵ und R¹⁶ eine Nitrogruppe, eine Cyanogruppe oder eine Sulfonatgruppe bedeutet, und die übrigen Reste R¹² bis R¹⁶ unabhängig voneinander Wasserstoff, Chlor, eine Hydroxygruppe, eine C₁- bis C₅-Hydroxyalkylgruppe eine C₁- bis C₅-Alkoxygruppe, eine C₂- bis C₅-Hydroxyalkoxygruppe oder eine Carboxylgruppe darstellen,
(3) mehrkernigen aromatischen Heterozyklen oder Isozyklen, die mit mindestens einer Amino- oder Hydroxygruppe und mindestens einer Nitrogruppe, Cyanogruppe oder Sulfonatgruppe substituiert sind,
(4) fünf- bis sechsgliedrigen Heterozyklen mit 1 bis 3 Stickstoffatomen, die mit mindestens einer Amino- oder Hydroxygruppe und mindestens einer Nitrogruppe, Cyanogruppe oder Sulfonatgruppe substituiert sind,
(5) offenkettigen gesättigten oder ungesättigten Verbindungen, die mindestens zwei Aminogruppen oder je eine Aminogruppe und eine Hydroxygruppe sowie mindestens eine Nitrogruppe, Cyanogruppe oder Sulfonatgruppe aufweisen.

3. Mittel nach Anspruch 1 oder 2, dadurch gekennzeichnet, daß die Verbindung der Formel (II) ausgewählt ist aus 2-Nitroanilin, 3-Nitroanilin, 4-Nitro-o-phenylendiamin, 2-Amino-5-nitrophenol, 1,4-Diamino-2-nitrobenzol, 2-Amino-3,5-dinitrophenol, Pikraminsäure, 1,2-Diamino-3-nitrobenzol, 2-Cyanoanilin, Diaminomaleinsäuredinitril, 1-Amino-2-naphthol-4-sulfonsäure, 2-Aminophenol-4-sulfonsäure, 2-(4,5,6-Trihydroxy-3-oxo-3H-xanthen-9-yl)-benzolsulfonäure und p-Phenylendiamin-sulfonsäure.

4. Mittel nach einem der Ansprüche 1 bis 3, dadurch gekennzeichnet, daß das o-Benzochinon der Formel (I) ausgewählt ist aus 4,5-Dimethoxy-1,2-benzochinon, 4,5-Methylendioxy-1,2-benzochinon, 4,5-Dianilino-1,2-benzochinon, o-Chloranil und 3,5-Di-(tert-butyl)-1,2-benzochinon.

5. Mittel nach einem der Ansprüche 1 bis 4, dadurch gekennzeichnet, daß das o-Benzochinon der Formel (I) in einer Menge von 0,02 bis 3 Gewichtsprozent enthalten ist.

6. Mittel nach einem der Ansprüche 1 bis 5, dadurch gekennzeichnet, daß die Verbindung der Formel (II) in einer Menge von 0,03 bis 6 Gewichtsprozent enthalten ist.

7. Mittel nach einem der Ansprüche 1 bis 6, dadurch gekennzeichnet, daß es einen pH-Wert von 3 bis 10,5 aufweist.

8. Mittel nach einem der Ansprüche 1 bis 7, dadurch gekennzeichnet, daß es ein Mittel zum Färben menschlicher Haare ist.

9. Mittel nach einem der Ansprüche 1 bis 8, dadurch gekennzeichnet, daß es in Form eines 2-Komponentenpräparates vorliegt, bei dem die Verbindungen der Formel (I) und Formel (II) getrennt voneinander abgepackt sind.

10. Verfahren zum Färben von menschlichen Haaren, dadurch gekennzeichnet, daß man ein Haarfärbemittel nach einem der Ansprüche 1 bis 9 in einer für die Haarfärbung ausreichenden Menge auf das Haar aufbringt und das Haar nach einer Einwirkungszeit von 20 bis 30 Minuten bei 20 bis 40 Grad Celsius mit Wasser ausspült und sodann trocknet.

11. Verfahren zum Färben von menschlichen Haaren, dadurch gekennzeichnet, daß man die Haare unter Verwendung eines Mittels gemäß Anspruch 9 färbt, wobei die die Verbindung der Formel (I) enthaltende Zubereitung und die die Verbindung der Formel (II) enthaltende Zubereitung in einem Zeitabstand von 2 bis 5 Minuten nacheinander auf das Haar aufgetragen werden, sodann nach einer Einwirkungszeit von 20 bis 40 Minuten bei 20 bis 40 Grad Celsius das Haar mit Wasser ausgespült und anschließend getrocknet wird.
